# EUROPÄISCHE PATENTSCHRIFT

(11) **EP 0 556 546 B1**
(45) Veröffentlichungstag und Bekanntmachung des Hinweises auf die Patenterteilung: **18.10.2000**
(21) Anmeldenummer: 93100183.8
(22) Anmeldetag: 08.01.1993
(51) Int. Cl.: A61K 7/32, A61K 7/04, A61K 33/06, A61K 33/30

(54) **Viruzides Desinfektionsmittel**
Viricidal disinfecting composition
Composition desinfectante virucide

(30) Priorität: 10.01.1992 DE 4200499
(43) Veröffentlichungstag der Anmeldung: 25.08.1993
(73) Patentinhaber: Bode Chemie GmbH & Co., D-22525 Hamburg (DE)
(72) Erfinder: Widulle,Herbert Dr., W-2000 Hamburg 53 (DE); Pietsch,Hanns Dr., W-2000 Hamburg 13 (DE); Steinmann,Jochen Dr., W-2000 Bremen 33 (DE); Meyer,Brigitte, W-2000 Hamburg 60 (DE); Stumpe,Rosemarie, W-2000 Hamburg 50 (DE); Ostermeyer,Christiane, W-2000 Hamburg 56 (DE)
(74) Vertreter: Dinné, Erlend

(56) Entgegenhaltungen:
- EP-A- 0 012 115
- EP-A- 0 251 303

## Beschreibung

Die Erfindung betrifft ein alkoholhaltiges Desinfektionsmittel, das insbesondere zur Behandlung der Hände und der Haut geeignet ist und sich durch eine besonders gute Wirksamkeit gegen Viren auszeichnet.

Aus den europäischen Patentanmeldungen 176 720 und 251 303 sind bereits alkoholhaltige viruzide Mittel bekannt. So ist in der EP-A-251 303 die Kombination von mindestens 70 Gew.-% Ethanol oder Propanolen mit 0,5 - 5 Gew.-% einer kurzkettigen organischen Säure bekannt. Diese Mittel zeigen aber in der Praxis nicht immer eine zufriedenstellende Wirkung.

Weiterhin sind alkoholische Lösungen von Zinkchlorid (Römpps Chemie-Lexikon, 1988, Seite 4709) und die Löslichkeit von Aluminiumchlorid-Hexahydrat in Alkohol bekannt (CRC Handbook of Chemistry and Physics, 1980, B-51).

Auch die geringe Eignung einiger Schwermetallsalze als antibakterielle Wirkstoffe ist beschrieben (Kirk-Othmer, Encyclopedia of Chemical Technology, Volume 7 (1979), 806).

Aufgabe der Erfindung ist es daher, alkoholhaltige Desinfektionsmittel mit verbesserter Wirkung, vorzugsweise gegen Viren, insbesondere gegen nackte (hüllenlose) Viren zu schaffen.

Diese Aufgabe wird gelöst durch die Verwendung mindestens einer Lewis-Säure zur Herstellung eines alkoholhaltigen Desinfektionsmittels, das mindestens 60 Gew.-%, bezogen auf das Gesamtgewicht des Mittels, mindestens eines Alkoholes enthält.

Gut geeignet sind z.B. monofunktionale, niedrigmolekulare Alkohole, insbesondere Alkanole mit 1 bis 4 Kohlenstoffatomen. Bevorzugt werden Methanol, Ethanol, Isopropanol oder n-Propanol verwendet.

Besonders bevorzugt wird Ethanol. Bevorzugt sind auch Gemische der Alkohole, z.B. mit zwei oder drei Alkoholen, insbesondere solche, die Ethanol enthalten.

Bevorzugte Gemische der Alkohole sind Ethanol/n-Propanol, Ethanol/iso-Propanol und iso-Propanol/n-Propanol, wobei für solche Gemische folgende Gewichtsprozente bevorzugt sind: der Anteil des Alkohols mit einem höheren Siedepunkt beträgt 10 bis 50 Gewichtsprozent und der Anteil des Alkohols mit niedrigerem Siedepunkt beträgt 60 bis 20 Gewichtsprozent, jeweils bezogen auf das gesamte Mittel.

Erfindungsgemäß beträgt der Alkoholgehalt mindestens 60 Gew.-%, vorzugsweise aber mindestens 70 Gew.-%, besonders bevorzugt aber mindestens 80 Gew.-%, jeweils bezogen auf das Gewicht des gesamten Mittels. Mischungen, die ausschließlich einen oder mehrere Alkohole und eine oder mehrere Lewis-Säuren enthalten, werden auch bevorzugt.

Vorteilhafterweise beträgt der Alkoholgehalt 75 bis 95 Gew.-%, insbesondere aber 80 bis 90 Gew.-%, jeweils bezogen auf das gesamte Mittel.

Geeignete Lewis-Säuren sind beispielsweise Salze, z.B. Halogenide, wie Fluoride, Bromide oder insbesondere Chloride von Bor, Aluminium, Phosphor, Zirkon, Eisen, Zink oder Zinn oder Aluminium-Zirkonium-Salze. Besonders bevorzugt werden Salze, insbesondere Halogenide, vorzugsweise Chloride von Aluminium, Eisen, Zink oder Zinn. Als besonders vorteilhaft haben sich die Chloride von Aluminium, Eisen, Zink oder Zinn erwiesen, insbesondere aber die Chloride des Aluminiums, insbesondere AlCl₃, oder Zinks, insbesondere ZnCl₂ und deren Hydrate oder Aquo-Komplexe.

Auch Mischungen der Lewis-Säuren können eingesetzt werden, ebenso wie gemischte Komplexe der genannten Komponenten wie z.B. gemeinsame Hydrate, Chlorohydrate oder Chloride, beispielsweise solche mit Aluminium, Zirkon oder Zink.

Mischungen können beispielsweise aus zwei oder drei Komponenten bestehen, vorzugsweise aber aus zwei Komponenten.

Bevorzugt sind Gemische von Aluminiumchlorid und Aluminiumhydroxychlorid, wobei das Mischungsverhältnis vom reinen Aluminiumchlorid bis zum reinen Aluminiumhydroxychlorid variieren kann.

Besonders bevorzugte Gemische sind Aluminiumchlorid/Aluminiumhydroxychlorid in den folgenden Gewichts-Verhältnissen 19:1, 8:14, 5:23 oder 1:19.

Bevorzugt werden die folgenden Lewis-Säuren und deren Gemische, insbesondere mit 2 oder 3 Komponenten:
Aluminiumchlorid
Dialuminiumchlorohydrat
Aluminiumzirkoniumchlorohydrate
Aluminium sulfat
Aluminium acetat
Zinkchlorid
Zinksulfat
Aluminium-hydroxychloride
Aluminium-zirkoniumchlorid
Aluminium-Zirkoniumsulfat
Aluminium-nitrat
Aluminium-lactat
Aluminium-allantoinat
Eisen-III-chlorid.

Die Lewis-Säuren bzw. deren das Gemische werden dem Desinfektionsmittel z.B. in einer Menge von 0,05 Gew.-% bis 5 Gew.-%, vorzugsweise 0,1 Gew.-% bis 2,0 Gew.-%, insbesondere aber 0,3 Gew.-% bis 1,5 Gew.-% zugesetzt, jeweils bezogen auf das gesamte Mittel.

Bevorzugt sind Desinfektionsmittel, die zwei oder mehr Lewissäuren gleichzeitig enthalten.

Bevorzugt wird auch ein Desinfektionsmittel, das mindestens 65 Gew.-% Ethanol und 0,1 bis 3,0 Gew.-% Aluminiumchlorid Hexahydrat enthält sowie ein Desinfektionsmittel, das mindestens 75 Gew.-% Propanole und 0,05% bis 2,0 Gew.-% Aluminiumchlorid enthält, jeweils bezogen auf das gesamte Mittel.

Die erfindungsgemäßen Desinfektionsmittel können weiterhin Wasser enthalten. Ein Zusatz von Wasser zu der Metallsalz-Alkohollösung ist bis zu einem Gehalt von 60 Gew.-% Alkohol des genannten Mittels nicht schädlich für die Wirksamkeit, kann aber die Löslichkeit der Lewis-Säuren schon in geringen Mengen deutlich erhöhen und daher in Einzelfällen zweckmäßig sein. Andererseits ist hinsichtlich der Wirksamkeit kein Wasserzusatz erforderlich. Der Wassergehalt der Desinfektionsmittel kann daher etwa 0 bis 30 Gew.-%, insbesondere 8 bis 20 Gew.-% betragen, jeweils bezogen auf das gesamte Mittel.

Weiterhin können die erfindungsgemäßen Desinfektionsmittel auch Remanenzwirkstoffe enthalten, beispielsweise Chlorhexidindigluconat, Mecetroniumetilsulfat, Jod, Benzalkoniumchlorid, Wasserstoffperoxid oder andere Peroxide, Peressigsäure oder andere Persäuren oder Phenole wie Tetrabrommethylphenol oder o-Phenylphenol oder Gemische davon. Bevorzugte Mengen sind 0,025 bis 5 Gewichtsprozent.

Die erfindungsgemäßen Desinfektionsmittel können auch einen oder mehrere Hautpflegestoffe, insbesondere Rückfetter enthalten. Bevorzugt sind Paraffinium subliquidum, Isostearylisostearat, Glycerin, Allantoin, Isopropylmyristat, Isopropylpalmitat oder Milchsäure oder Gemische davon.

Diese Hautpflegestoffe können z.B. in Mengen von 0,1 - 3 Gewichtsprozent enthalten sein.

Außerdem können in dem erfindungsgemäßen Mittel weitere Hilfsstoffe, z.B. Farbstoffe, Färbemittel für die Haut oder Parfüm enthalten sein.

Auch der Zusatz von Netzmitteln oder Tensiden, wie z.B. Ethercarbonsäuren, Fettalkoholethoxylaten, an sich bekannten Amphotensiden, z.B. in Mengen von 0,1 bis 3,0 Gew.-% ist geeignet.

Bevorzugte erfindungsgemäße Desinfektionsmittel haben folgende Zusammensetzung:

| | Gewichtsprozent |
|---|---|
| Alkohol | 60 bis 95 |
| Lewis-Säure | 0,2 bis 1,5 |
| Wasser | 0 bis 25 |
| Rückfetter | 1 bis 3 |
| Remanenzwirkstoffe und Hilfsstoffe und Netzmittel | 0 bis 2 |

Die Herstellung der erfindungsgemäßen Mittel erfolgt in an sich bekannter Weise. Zu der gewünschten Wasser- und/oder Alkoholmenge wird die Lewis-Säure gegeben und gelöst, gegebenenfalls unter Rühren und Erwärmen. Dann werden weitere Inhaltsstoffe unter Rühren zugegeben. Auch dabei kann es hilfreich sein, die Temperaturen bis auf etwa 25°C zu erhöhen. Gegenstand der Erfindung ist auch das Herstellungsverfahren.

Überraschenderweise zeichnen sich die erfindungsgemäßen Desinfektionsmittel durch eine hohe viruzide Breitbandwirkung insbesondere gegenüber hüllenlosen (nackten) Viren aus, wie Untersuchungen mit Polioviren ergaben. Es zeigte sich, daß die zugesetzten Lewis-Säuren die viruzide Wirkung von Alkoholen stark erhöhen oder erst in ausreichendem Maß bewirken. Die Lewis-Säuren, insbesondere Aluminiumchlorid und/oder Zinkchlorid vervielfachen dabei die an sich bekannte virusinaktivierende Wirksamkeit von Alkoholen, z.B. Ethanol oder Propanol. Da Lewis-Säuren allein gegenüber Viren keine ausgesprochene Wirkung zeigen, bewirkt offensichtlich ein starker Synergismus die unerwartete Wirksamkeit der Kombination dieser Stoffe. Sogar Alkohole wie Propanole, die allein auch in hoher Konzentration keine zufriedenstellende Wirkung haben, erlangen in der erfindungsgemäßen Kombination eine hervorragende viruzide Wirkung.

Die erfindungsgemäßen viruziden Desinfektionsmittel wirken unerwartet stark und schnell inaktivierend gegenüber Polioviren (Typ 1, Stamm Mahoney) und übertreffen darin die schon aus dem EP-A-251 303 bekannten Mittel mit speziellen Carbonsäuren, die eine Zeit von 1 bis 2 Minuten für eine Minderung der Infektiösität um mindestens 4 lg-Stufen benötigen, wie aus dem Vergleichsversuch ersichtlich ist.

Gegenstand der Erfindung ist daher auch die Verwendung des erfindungsgemäßen Desinfektionsmittels oder der erfindungsgemäßen Kombination von Alkohol und Lewis-Säure als viruzides Mittel, insbesondere mit Breitbandwirkung sowie die Verwendung der vorstehend genannten Mittel und Kombination zur Herstellung eines viruziden Mittels, insbesondere mit Breitbandwirkung.

Die erfindungsgemäßen Desinfektionsmittel können als Hautdesinfektionsmittel, Händedesinfektionsmittel und als Desinfektionsmittel für insbesondere unbelebte Oberflächen oder insbesondere harte Oberflächen und Geräte verwendet werden.

Die erfindungsgemäßen Desinfektionsmittel können durch wiederholtes Einreiben in die Haut der Hände und Unterarme, durch Auftragen mit einem Tupfer auf die Körperhaut oder Aufsprühen angewendet werden.

Die erfindungsgemäßen Desinfektionsmittel eignen sich besonders für die folgenden Zwecke:
präoperative Händedesinfektion des Personals, Händedesinfektion zur Unterbrechung von Infektionsketten, Hautdesinfektion vor Operationen, Punktionen, Injektionen oder sonstigen Wundsetzungen.

Alle Prozentangaben oder Teile beziehen sich, soweit nicht anders angegeben, auf das Gewicht, insbesondere auf das Gesamtgewicht der Zubereitungen.

Die folgenden Beispiele dienen zur Erläuterung der Erfindung.

Aus den folgenden Bestandteilen wird ein Desinfektionsmittel hergestellt.

### Beispiel 1

| Inhaltsstoffe | Gew.-% |
|---|---|
| Glycerin | 0,50 |
| Propanol-1 | 30,00 |
| Propanol-2 | 45,00 |
| Paraffinum subliquidum | 0,75 |
| Aluminiumchlorid-Hexahydrat | 0,54 |
| Wasser | ad 100,00 |

Zur Herstellung werden Wasser und/oder Alkohole vorgelegt. Dann werden die weiteren Bestandteile unter Rühren zugefügt.

### Beispiel 2

| Inhaltsstoffe | Gew.-% |
|---|---|
| Ethanol | 80,00 |
| Aluminiumchlorid x 6H₂O | 0,90 |
| Wasser | ad 100 |

Die Herstellung erfolgt wie vorstehend angegeben.

### Beispiel 3

| Inhaltsstoffe | Gew.-% |
|---|---|
| Ethanol | 95,00 |
| Aluminiumchlorid x 6H₂O | 1,00 |
| Wasser | 4,00 |

Die Herstellung erfolgt wie vorstehend angegeben.

Die Herstellung der Mittel der folgenden Beispiele erfolgt wie vorstehend angegeben.

Zur Beurteilung der viruziden Wirkung wird entsprechend den Richtlinien des Bundesgesundheitsamtes und der Deutschen Vereinigung zur Bekämpfung der Viruskrankheiten (Bundesgesundheitsblatt 25, Nr. 12, Dezember 1982) die Titerreduktion (Zahlenangabe in lg 10 nach 0,5 Minuten, einer Minute bzw. 2 Minuten und nach 5 Minuten Behandlungszeit) für Poliovirus Typ I, Stamm Mahoney, ermittelt.

### Beispiel 4

| Inhaltsstoffe | Gew.-% | Titerreduktion | |
|---|---|---|---|
| | | 1 min. | 5 min. |
| Ethanol | 80,00 | über 5,62 | über 5,62 |
| Aluminiumchlorid x 6H₂O | 0,90 | | |
| Wasser | ad 100 | | |

### Beispiel 5

| Inhaltsstoffe | Gew.-% | Titerreduktion |
|---|---|---|
| | | 0,5 min. |
| Ethanol | 80,00 | über 6 |
| Aluminiumchlorid x 6H₂O | 2,00 | |
| Wasser | ad 100 | |

### Beispiel 6

| Inhaltsstoffe | Gew.-% | Titerreduktion |
|---|---|---|
| | | 2 min. |
| 1-Propanol | 30,00 | |
| 2-Propanol | 45,00 | 4,5 ohne Eiweißbelastung |
| Mecetroniumetilsulfat | 0,20 | über 5,25 mit Eiweißbelastung |
| Aluminiumchlorid x 6H₂O | 2,0 | |
| Dialuminiumchlorohydrat | 0,8 | |
| Wasser | ad 100 | |

Die Beispiele 4, 5 und 6 zeigen, daß bereits mit geringen Mengen Lewis-Säuren eine bessere Wirksamkeit erreicht wird, als bei der Kombination mit organischen Säuren, wie in EP-A-251 303, Beispiele 1 bis 6, beschrieben.

### Beispiel 7

Es wurde wie in Beispiel 6 verfahren, nur wurde statt Aluminiumchlorid und Dialuminiumchlorohydrat als Lewis-Säure Aluminium-Zirkoniumchlorid eingesetzt.

### Beispiel 8

| Inhaltsstoffe | Gew.-% |
|---|---|
| Ethanol | 99,6 |
| Aluminiumchlorid x 6H₂O | 0,4 |

### Beispiel 9

| Inhaltsstoffe | Gew.-% |
|---|---|
| Ethanol | 60 |
| Aluminiumchlorid x 6H₂O | 4 |
| Wasser | ad 100 |

### Beispiel 10

Es wurde wie in Beispiel 4 verfahren, nur wurde statt Aluminiumchlorid-Hexahydrat Zinkchlorid (ZnCl₂) verwendet.

## Patentansprüche

1. Verwendung mindestens einer Lewis-Säure zur Herstellung eines alkoholhaltigen Desinfektionsmittels, das mindestens 60 Gew.-%, bezogen auf das Gesamtgewicht des Mittels, mindestens eines Alkoholes enthält.

2. Verwendung gemäß Anspruch 1, dadurch gekennzeichnet, daß als Alkohol Ethanol, Isopropanol oder n-Propanol oder deren Gemische verwendet werden.

3. Verwendung gemäß Anspruch 1, dadurch gekennzeichnet, daß als Lewis-Säure Salze des Aluminiums oder Zinks verwendet werden.

4. Verwendung gemäß Anspruch 1, dadurch gekennzeichnet, daß der Anteil der Lewis-Säuren 0,05 bis 5 Gew.-% des Mittels beträgt, jeweils bezogen auf das gesamte Mittel.

5. Verwendung gemäß Anspruch 1, dadurch gekennzeichnet, daß als Lewis-Säure Aluminiumchlorohydrate, Aluminiumchlorid (AlCl₃) oder Zinkchlorid (ZnCl₂) verwendet werden.

6. Verwendung gemäß Anspruch 1, dadurch gekennzeichnet, daß zwei oder mehr Lewis-Säuren gleichzeitig verwendet werden.

7. Verwendung gemäß Anspruch 1, dadurch gekennzeichnet, daß mindestens 65 Gew.-% Ethanol und 0,1 bis 3,0 Gew.-% Aluminiumchlorid-Hexahydrat verwendet werden, jeweils bezogen auf das gesamte Mittel.

8. Verwendung gemäß Anspruch 1, dadurch gekennzeichnet, daß mindestens 75 Gew.-% Propanole und 0,05% bis 2,0 Gew.-% Aluminiumchlorid verwendet werden, jeweils bezogen auf das gesamte Mittel.

9. Verwendung mindestens einer Lewis-Säure zur Herstellung eines alkoholhaltigen, viruziden Desinfektionsmittels, insbesondere mit Breitbandwirkung, das mindestens 60 Gew.-%, bezogen auf das Gesamtgewicht des Mittels, mindestens eines Alkoholes enthält.

10. Verwendung mindestens einer Lewis-Säure zur Herstellung eines alkoholhaltigen Desinfektionsmittels für die Hautdesinfektion, Händedesinfektion und für die Desinfektion von insbesondere unbelebten Oberflächen oder insbesondere harten Oberflächen und von Geräten, das mindestens 60 Gew.-%, bezogen auf das Gesamtgewicht des Mittels, mindestens eines Alkoholes enthält.

11. Verwendung gemäß Anspruch 1, dadurch gekennzeichnet, daß ein oder mehrere Hautpflegestoffe mitverwendet werden.

12. Verwendung gemäß Anspruch 1, dadurch gekennzeichnet, daß
| | Gew.-% |
|---|---|
| 1-Propanol | 30,00 |
| 2-Propanol | 45,00 |
| Mecetroniumetilsulfat | 0,20 |
| Aluminiumchlorid x 6H₂0 | 2,00 |
| Dialuminiumchlorohydrat | 0,80 |
| Wasser | ad 100 |
verwendet werden.

## Claims

1. Use of at least one Lewis acid for the preparation of an alcohol-containing disinfectant which comprises at least 60% by weight, based on the total weight of the composition, of at least one alcohol.

2. Use according to Claim 1, characterized in that the alcohol used is ethanol, isopropanol or n-propanol or mixtures thereof.

3. Use according to Claim 1, characterized in that the Lewis acid used is a salt of aluminium or zinc.

4. Use according to Claim 1, characterized in that the proportion of Lewis acids is from 0.05 to 5% by weight of the composition, in each case based on the total composition.

5. Use according to Claim 1, characterized in that the Lewis acid used is an aluminium chlorohydrate, aluminium chloride (AlCl₃) or zinc chloride (ZnCl₂).

6. Use according to Claim 1, characterized in that two or more Lewis acids are used at the same time.

7. Use according to Claim 1, characterized in that at least 65% by weight of ethanol and from 0.1 to 3.0% by weight of aluminium chloride hexahydrate are used, in each case based on the total composition.

8. Use according to Claim 1, characterized in that at least 75% by weight of propanols and from 0.05% to 2.0% by weight of aluminium chloride are used, in each case based on the total composition.

9. Use of at least one Lewis acid for the preparation of an alcohol-containing virucidal disinfectant, in particular having broad-band action, which comprises at least 60% by weight, based on the total weight of the composition, of at least one alcohol.

10. Use of at least one Lewis acid for the preparation of an alcohol-containing disinfectant for skin disinfection hand disinfection and for the disinfection of, in particular, inanimate surfaces or, in particular, hard surfaces and of apparatuses, which comprises at least 60% by weight, based on the total weight of the composition, of at least one alcohol.

11. Use according to Claim 1, characterized in that one or more skin care substances are co-used.

12. Use according to Claim 1, characterized in that
| | % by weight |
|---|---|
| 1-propanol | 30.00 |
| 2-propanol | 45.00 |
| mecetronium ethylsulphate | 0.20 |
| aluminium chloride × 6H₂O | 2.00 |
| dialuminium chlorohydrate | 0.80 |
| water | ad 100 |
are used.

## Revendications

1. Utilisation d'au moins un acide de Lewis pour la préparation d'une composition désinfectante contenant de l'alcool, qui comprend au moins 60% en poids, par rapport au poids total de la composition, d'au moins un alcool.

2. Utilisation selon la revendication 1, caractérisée en ce que l'on utilise, en tant qu'alcool, l'éthanol, l'isopropanol ou le n-propanol, ou leurs mélanges.

3. Utilisation selon la revendication 1, caractérisée en ce que l'on utilise des sels d'aluminium ou de zinc en tant qu'acide de Lewis.

4. Utilisation selon la revendication 1, caractérisée en ce que la proportion des acides de Lewis est de 0,05 à 5% en poids de la composition, dans chaque cas par rapport à la composition totale.

5. Utilisation selon la revendication 1, caractérisée en ce que l'on utilise des chlorhydrates d'aluminium, le chlorure d'aluminium (AlCl₃) ou le chlorure de zinc (ZnCl₂) en tant qu'acide de Lewis.

6. Utilisation selon la revendication 1, caractérisée en ce que l'on utilise simultanément deux acides de Lewis ou plus.

7. Utilisation selon la revendication 1, caractérisée en ce que l'on utilise au moins 65% en poids d'éthanol et de 0,1 à 3,0% en poids d'hexahydrate de chlorure d'aluminium, dans chaque cas par rapport à la composition totale.

8. Utilisation selon la revendication 1, caractérisée en ce que l'on utilise au moins 75% en poids de propanols et de 0,05% à 2,0% en poids de chlorure d'aluminium, dans chaque cas par rapport à la composition totale.

9. Utilisation d'au moins un acide de Lewis pour la préparation d'une composition désinfectante virucide contenant de l'alcool, en particulier avec une large plage d'action, qui comprend au moins 60% en poids, par rapport au poids total de la composition, d'au moins un alcool.

10. Utilisation d'au moins un acide de Lewis pour la préparation d'une composition désinfectante contenant de l'alcool, pour la désinfection de la peau, la désinfection des mains et pour la désinfection en particulier de surfaces inanimées ou en particulier de surfaces dures, et d'appareils, laquelle comprend au moins 60% en poids, par rapport au poids total de la composition, d'au moins un alcool.

11. Utilisation selon la revendication 1, caractérisée en ce que l'on utilise conjointement une ou plusieurs substances pour les soins de la peau.

12. Utilisation selon la revendication 1, caractérisée en ce que l'on utilise
| | % en poids |
|---|---|
| 1-propanol | 30,00 |
| 2-propanol | 45,00 |
| Mécétroniuméthylsulfate | 0,20 |
| Chlorure d'aluminium × 6H₂O | 2,00 |
| Chlorhydrate de dialuminium | 0,80 |
| Eau | qsp 100 |
